# EUROPÄISCHE PATENTANMELDUNG

(11) **EP 0 706 823 A1**
(43) Veröffentlichungstag der Anmeldung: **17.04.1996**
(21) Anmeldenummer: 95114110.0
(22) Anmeldetag: 08.09.1995
(51) Int. Cl.: B01J 29/06, C07C 17/358

(54) **Zeolith-Katalysatoren mit verbesserter Regenerierbarkeit**

(30) Priorität: 30.09.1994 DE 4434981; 30.09.1994 DE 4434980
(71) Anmelder: HOECHST AKTIENGESELLSCHAFT, D-65929 Frankfurt am Main (DE)
(72) Erfinder: Neuber, Marita, Dr., D-60528 Frankfurt am Main (DE); Roscher, Günter, Dr., D-65779 Kelkheim (DE)

(57) **Zusammenfassung**

Die Erfindung betrifft Katalysatoren, die Zeolithe und Oxide oder Salze der Elemente der 2. und 3. Hauptgruppe des Periodensystems enthalten und eine verbesserte Regenerierbarkeit aufweisen, sowie ihre Verwendung für die Umsetzung halogenierter Aromaten.

## Beschreibung

Gegenstand der vorliegenden Erfindung sind Katalysatoren, die Zeolithe und Oxide oder Salze der Elemente der 2. und 3. Hauptgruppe des Periodensystems enthalten und eine verbesserte Regenerierbarkeit aufweisen, sowie ihre Verwendung für die Umsetzung halogenierter Aromaten.

Zeolithe werden häufig als saure Katalysatoren für die Umsetzung von Aromaten verwendet. Sie zeichnen sich durch eine hohe Säurestärke und dadurch hohe Aktivität aus, die es erlaubt, auch stark desaktivierte Aromaten wie z.B. ein- oder mehrfach halogenierte Aromaten durch Isomerisierung oder Alkylierung umzusetzen.

Darüber hinaus lassen sie sich im Vergleich zu herkömmlichen Friedel-Crafts-Katalysatoren als Feststoffe und aufgrund ihrer Ungiftigkeit einfach handhaben und sie ermöglichen eine einfache Abtrennung des Reaktionsprodukts vom Katalysator. Zudem sind sie im Vergleich zu anderen festen Säuren wie z.B. Pillared Clays chemisch und thermisch sehr stabil.

Bei sauer katalysierten Umsetzungen von Aromaten bilden sich in Nebenreaktionen hochmolekulare, kohlenstoffreiche Verbindungen, die sich auf dem Katalysator ablagern. Diese Verbindungen, kurz "Koks" genannt, blockieren den Zugang der Reaktanden zu den aktiven Zentren und vermindern die Aktivität des Zeoliths. Die Geschwindigkeit, mit der der Katalysator desaktiviert, ist von vielen Einflußfaktoren wie z.B. der Art der untersuchten Reaktion, den Reaktanden, dem Zeolithtyp, der chemischen Zusammensetzung des Zeoliths oder den Versuchsbedingungen abhängig (siehe z.B. M. Guisnet und P. Magnoux, NATO ASI Ser., Ser. C 352 (1992) 457/474).

Durch die Entfernung der Koksablagerungen kann der Katalysator wieder regeneriert werden.

In der Regel genügt ein Abbrennen mit Luft oder Luft-/Stickstoffverbindungen bei Temperaturen bis ca. 600°C. Die Reaktion ist stark exotherm. Um Temperaturspitzen zu vermeiden, die den Katalysator thermisch schädigen könnten, wird nach dem Stand der Technik mit Luft-/Stickstoffgemischen mit sehr geringen Sauerstoffkonzentrationen begonnen und die Sauerstoffkonzentration im Verlauf der Reaktion nach und nach erhöht. Daneben wurden auch Regenerierverfahren beschrieben, bei denen andere oxidierende Verbindungen zur Entfernung des Kokses eingesetzt werden.

In vielen Arbeiten wird beschrieben, daß der Zeolith-Katalysator bei geeigneten Bedingungen durch die Regenerierung nicht verändert wird und seine ursprüngliche Aktivität wieder erreicht. Koksbildung und -entfernung sind demnach vollständig reversible Prozesse.

Bei der Umsetzung verschiedener halogenierter, insbesondere fluorierter Aromaten an Zeolith-Katalysatoren wurde nun gefunden, daß die Katalysatoren nur sehr eingeschränkt regenerierbar sind. So verlieren manche Zeolithe schon nach einer einzigen Regenerierung einen Großteil ihrer Aktivität. Oft werden auch die Selektivitäten zu einzelnen Reaktionsprodukten verändert.

Eine verbesserte Regenerierbarkeit von Zeolith-Katalysatoren für sauer katalysierte Umsetzungen von halogenierten, insbesondere fluorierten Aromaten erhöht die Wirtschaftlichkeit dieser Verfahren. Es bestand somit ein Bedarf sowohl nach Zeolith-Katalysatoren, die eine derartige verbesserte Regenerierbarkeit aufweisen als auch nach einem Verfahren, das zu einer verbesserten Regenerierbarkeit von Zeolith-Katalysatoren führt, die für die genannten Umsetzung eingesetzt werden.

Diese Aufgabe wird dadurch gelöst, daß Oxide oder Salze der Elemente der zweiten und dritten Hauptgruppe des Periodensystems der Elemente oder Mischungen daraus zum Zeolith zugesetzt werden. Hierdurch entsteht ein neuer Katalysator, dessen Regenerierbarkeit deutlich verbessert ist.

Als Beispiele für geeignete Oxide oder Salze seien CaO, MgO, Al₂O₃, B₂O₃, CaCO₃ oder MgCO₃ genannt, wobei sich CaO, CaCO₃ und Al₂O₃ besonders gut eignen. Es können auch Mischungen der genannten Verbindungen eingesetzt werden.

Die genannten Verbindungen werden einfach physikalisch mit dem Zeolith gemischt. Durch dieses Verfahren werden die erfindungsgemäßen Katalysatoren hergestellt. Um eine gute Vermischung zu erhalten sollten sich die Korngrößen von Zeolith und Zusatz nicht sehr stark unterscheiden.

Da die Reaktion in der Regel in einem Festbettreaktor durchgeführt wird, ist der Zeolith meist mit einem Binder verpreßt und in Form von Tabletten, Pellets oder Strängen vorgeformt. Wenn die zugesetzten Verbindungen als stückige Feststoffe anfallen, so können sie auf eine vergleichbare Größe wie die Zeolithformlinge gebracht und direkt mit ihnen vermischt werden. Fallen die zugesetzten Verbindungen als Pulver an, so können sie ähnlich wie der Zeolith mit einem geeigneten Bindermaterial verpreßt und zu Strängen, Pellets oder Tabletten geeigneter Größe geformt werden. Als Bindermaterialien eignen sich prinzipiell dieselben Materialien wie für die Zeolithverformung, beispielsweise SiO₂, Al₂O₃ oder TiO₂.

Die erfindungsgemäßen Katalysatoren enthalten vorteilhaft zwischen 5 und 150 Vol-% der Zusatzstoffe bezogen auf die Zeolithschüttung. In vielen Fällen bewährt haben sich Katalysatoren, die zwischen 20 und 70 Volumen-% an Oxiden oder Salzen bezogen auf das Volumen der Zeolithschüttung enthalten.

Zu den Zeolithen, deren Regenerierbarkeit durch den Zusatz von Oxiden oder Salzen der Elemente der zweiten und dritten Hauptgruppe verbessert wurden, gehören alle Zeolithtypen, die für die Umsetzungen halogenierter Aromaten eingesetzt werden können, insbesondere solche, die sich durch eine hohe Säurestärke auszeichnen. Solche Zeolithe sind beispielsweise Mordenit, Beta, ZSM-5, ZSM-11, ZSM-12, ZSM-48, EU-1 oder Y-Zeolithe.

Die Regenerierung der Katalysatoren kann mit Luft oder Luft-/Stickstoffgemischen bei einer Temperatur von 450 bis 650°C, insbesondere 500 bis 600°C durchgeführt werden.

Große Bedeutung haben die erfindungsgemäßen Katalysatoren für die Umsetzung von halogenhaltigen Aromaten, insbesondere von ein- oder zweikernigen Aromaten, die mindestens ein und höchstens vier Halogenatome, insbesondere Fluoratome enthalten und durch einen oder mehrere Reste R, wobei R C₁- bis C₆-Alkyl, C₆- bis C₉-Cycloalkyl oder C₆- bis C₉-Phenyl bedeuten kann, substituiert sind.

Zu den katalysierten Reaktionen der genannten Aromaten gehören Isomerisierungen, Alkylierungen, Umlagerungen, Disproportionierungen und Crackreaktionen.

So wird beispielsweise durch den Zusatz von CaO zu einem HZSM-5-Zeolith ein Katalysator für die Isomerisierung von 2,4-Difluorbrombenzol hergestellt, der 10 mal ohne Abnahme von Aktivität und Selektivität regeneriert werden kann, während ein HZSM-5 ohne CaO schon nach einer einzigen Regenerierung einen deutlichen Abfall der Aktivität und auch der Selektivität zum erwünschten Isomeren zeigt.

Es war nicht zu erwarten, daß die erfindungsgemäßen Verbindungen im Vergleich zu Zeolithen ohne Zusatz deutlich verbesserte Regenerierbarkeit aufweisen.

Die genannten Verbindungen sind unter Regenerierbedingungen chemisch sehr stabil. Bei einer Reaktion zwischen Zeolith und Zusatz wäre aufgrund des oft basischen Charakters der Zusätze z.B. CaO an eine Neutralisation der sauren Zentren oder eine Verminderung der Säurestärke zu erwarten, was zu einer Abnahme der Aktivität führen würde. Überraschenderweise nimmt die Aktivität des Katalysators im Verlauf der Reaktion in manchen Fällen sogar zu.

Die verbesserte Regenerierbarkeit läßt sich auch nicht allein darauf zurückführen, daß mögliche, den Zeolith schädigende Verbindungen, die bei der Reaktion und der Regenerierung entstehen, wie z.B. Halogenwasserstoff und Wasser (unter Regenerierbedingungen) durch die Zusätze abgefangen werden. Es wurden in vergleichbaren Zeiträumen verminderte Konzentrationen an Halogenwasserstoff gefunden, wenn die genannten Zusätze anwesend waren. Die Laufzeiten waren in diesem Falle sehr viel länger im Vergleich zu den Versuchen ohne Zusätze und die insgesamt am Reaktorausgang gefundene Menge an Halogenwasserstoff war größer als im Vergleichsversuch. Beispielsweise wurde für die Isomerisierung von 2,4-Difluorbrombenzol 40 mmol HBr und 1 mmol HF während der gesamten Isomerisierungsdauer (21 h) gebildet gegenüber 120 mmol HBr und 5 mmol HF in Gegenwart von CaO innerhalb von 140 h bei gleicher Katalysatormenge. Dennoch war trotz der wesentlich größeren Menge an Halogenwasserstoff keine Verminderung der Aktivität des Katalysators festzustellen.

Bei einigen Reaktionen wurde sogar überraschenderweise festgestellt, daß durch Anwesenheit der genannten Zusätze sich die Desaktivierungsgeschwindigkeit vermindert und die Selektivität zum Zielprodukt erhöht, nachdem der Katalysator regeneriert wurde.

In den folgenden Beispielen soll der Gegenstand der Erfindung näher erläutert werden, ohne darauf beschränkt zu werden.

### Beispiele:

Die Versuche werden in einem Festbett-Strömungsreaktor bei Atmosphärendruck durchgeführt. Der Zeolith (80 % Zeolithanteil und 20 % Al₂O₃ als Binder, Korngröße 1 bis 2 mm) und Oxide oder Salze der Elemente der zweiten und dritten Gruppe des Periodensystems werden gemischt und in einen Quarzglasreaktor mit 20 mm Innendurchmesser gefüllt. Der Reaktor wird durch einen elektrischen Ofen beheizt. Der Katalysator wird vor der Reaktion bei 500°C in strömendem Stickstoff getrocknet. Der oder die Reaktanden werden von oben in den Reaktor dosiert und gegebenenfalls vor der Katalysatorschüttung verdampft. Am Reaktorausgang werden die Reaktionsprodukte kondensiert. Als Trägergas wird Wasserstoff verwendet. Der Trägergasstrom durch eine Waschflasche hinter dem Reaktor mit Wasser geleitet und die Konzentration von gebildetem Halogenwasserstoff im Waschwasser ermittelt. Die Reaktionsprodukte werden gaschromatographisch analysiert.

### Vergleichsbeispiel 1 und Beispiele 1 bis 3:

Für die folgenden Beispiele wurden jeweils 25 ml HSZM-5 als Zeolith eingesetzt und 12,5 ml der angegebenen Verbindung zugesetzt. Für Beispiel 2 wurden 80 % CaCO₃-Pulver mit 20 % Al₂O₃ als Binder verpreßt. Es wurden 12,5 ml/h 2,4-Difluorbrombenzol (DFBB) zudosiert und der Umsatz an 2,4-DFBB sowie die Selektivität zu 3,5-DFBB nach den angegebenen Zeiten bestimmt. Die Reaktionstemperatur betrug 320°C. Nach 14 bzw. 7 h Laufzeit wurde der Versuch abgebrochen und der Katalysator mit Luft regeneriert. Die Regeneriertemperatur betrug maximal 540°C. In der Tabelle 1 sind die Ergebnisse zusammengestellt.

**Tabelle 1**

| Beispiel | Zusatz | Katalysator | Zeit, h | Umsatz, mol-% | Selektivität, mol-% |
|---|---|---|---|---|---|
| V1 | ohne | frisch | 1 | 50 | 80 |
| | | | 14 | 24 | 86 |
| | | 1 x reg. | 1 | 42 | 81 |
| | | | 7 | 12 | 85 |
| 1 | CaO | frisch | 1 | 41 | 90 |
| | | | 14 | 22 | 90 |
| | | 1 x reg. | 1 | 57 | 88 |
| | | | 14 | 28 | 90 |
| | | 10 x reg. | 1 | 60 | 87 |
| | | | 14 | 38 | 90 |
| 2 | CaCO₃ | frisch | 1 | 34 | 90 |
| | | | 14 | 18 | 90 |
| | | 5 x reg. | 1 | 60 | 89 |
| | | | 14 | 35 | 90 |
| 3 | Al₂O₃ | frisch | 1 | 47 | 85 |
| | | | 14 | 36 | 91 |
| | | 2 x reg. | 1 | 55 | 88 |
| | | | 14 | 41 | 90 |

### Vergleichsbeispiel 2 und Beispiel 4:

Es wurden jeweils 25 ml HSZM-5 als Zeolith eingesetzt. Für Beispiel 4 wurden 12,5 ml der CaO zugesetzt. Anstelle von 2,4-Difluorbrombenzol wurden 12,5 ml/h 2-Fluortoluol zudosiert und der Umsatz nach den angegebenen zeiten bestimmt. Die Reaktionstemperatur wurde zwischen 320°C und 420°C variiert. Nach verschiedenen Laufzeiten wurde die Isomerisierung abgebrochen und der Katalysator mit Luft regeneriert. Die Regeneriertemperatur betrug maximal 540°C. In Tabelle 2 sind die Ergebnisse zusammengestellt.

**Tabelle 2**

| Beispiel | Zusatz | Katalysator | Zeit, h | Temp., °C | Umsatz, mol-% |
|---|---|---|---|---|---|
| V2 | ohne | frisch | 7 | 300 - 350 | 6 |
| | | 1 x reg. | 7 | 400 | 5 |
| | | 2 x reg. | 7 | 420 | 1 |
| 4 | CaO | frisch | 7 | 300 - 350 | 6 |
| | | 1 x reg. | 7 | 400 | 13 |
| | | 2 x reg. | 7 | 420 | 13 |

## Patentansprüche

1. Katalysatoren, die einen Zeolith und Oxide oder Salze der Elemente der zweiten und dritten Hauptgruppe des Periodensystems der Elemente oder Mischungen daraus enthalten.

2. Verbindungen nach Anspruch 1, die als Oxide oder Salze CaO, CaCO₃, Al₂O₃, B₂O₃ und MgO, insbesondere CaO, CaCO₃ oder Al₂O₃ enthalten.

3. Verbindungen nach den Ansprüchen 1 und 2, die zwischen 5 und 150 Vol-%, bevorzugt zwischen 20 und 70 Vol-%, der genannten Oxide und Salze bezogen auf das Volumen an Zeolith enthalten.

4. Verbindungen nach mindestens einem der Ansprüche 1 bis 3, dadurch gekennzeichnet, daß die Korngröße der eingesetzten Oxide und Salze der Korngröße der verpreßten Zeolithe entspricht.

5. Verbindungen nach mindestens einem der Ansprüche 1 bis 4, dadurch gekennzeichnet, daß die eingesetzten Oxide und Salze physikalisch mit dem Zeolith vermischt werden.

6. Verbindungen nach mindestens einem der Ansprüche 1 bis 5, dadurch gekennzeichnet, daß die eingesetzten Zeolithe ZSM-5, ZSM-11, ZSM-12, ZSM-48, Mordenit, Beta, EU-1 und Y sind.

7. Verfahren zur Regenerierung der Katalysatoren nach Anspruch 1 bis 6, dadurch gekennzeichnet, daß die Regenerierung mit Luft oder bevorzugt mit Luft-/Stickstoffgemischen durchgeführt wird.

8. Verfahren nach Anspruch 7, dadurch gekennzeichnet, daß die Regeneriertemperatur zwischen 450 und 650°C, bevorzugt zwischen 500 und 600°C liegt.

9. Verwendung von Katalysatoren nach mindestens einem der Ansprüche 1 bis 6 für die Umsetzung von halogenhaltigen Aromaten.

10. Verwendung nach Anspruch 9, dadurch gekennzeichnet, daß als halogenhaltige Aromaten ein- oder zweikernige Aromaten mit mindestens einem Halogenatom, die bis zu drei weitere Halogenatomen und/oder einen oder mehrere Reste R, wobei RC₁- bis C₆-Alkyl, C₆- bis C₉-Cycloalkyl oder C₆- bis C₉-Phenyl sein kann, enthalten können, eingesetzt werden.

11. Verwendung nach Anspruch 9 oder 10, dadurch gekennzeichnet, daß die umzusetzenden Aromaten mindestens ein Fluoratom enthalten.

12. Verwendung nach Anspruch 9 bis 11, dadurch gekennzeichnet, daß die Umsetzung eine Isomerisierungs-, Alkylierungs-, Umlagerungs-, Disproportionierungs- oder Crackreaktion ist.
